(19) Europäisches Patentamt

European Patent Office

Office européen des brevets

(11) Publication number: **0 316 320 B1**

(12) **EUROPEAN PATENT SPECIFICATION**

(45) Date of publication of patent specification: **03.11.93** (51) Int. Cl.⁵: **A61K 39/00, A61K 39/385**

(21) Application number: **87904658.9**

(22) Date of filing: **07.07.87**

(86) International application number:
**PCT/AU87/00204**

(87) International publication number:
**WO 88/00470 (28.01.88 88/03)**

(54) **REDUCTION OF REPRODUCTIVE LOSSES.**

(30) Priority: **11.07.86 AU 6855/86**

(43) Date of publication of application:
**24.05.89 Bulletin 89/21**

(45) Publication of the grant of the patent:
**03.11.93 Bulletin 93/44**

(84) Designated Contracting States:
**AT BE CH DE FR GB IT LI LU NL SE**

(56) References cited:
**AU-B- 6 668 981**
**AU-B- 6 686 481**
**GB-A- 2 166 951**

(73) Proprietor: **THE COMMONWEALTH SCIENTIFIC AND INDUSTRIAL RESEARCH ORGANIZATION**
**Limestone Avenue,**
**P.O. Box 1600**
**Canberra, Australian Capital Territory**
**2601(AU)**

(72) Inventor: **WILSON, Patricia, Ann**

**1 Yalanga Place**
**Lane Cove, NSW 2066(AU)**
Inventor: **COX, Ronald, Ian**
**14 Park Avenue**
**Beecroft, NSW 2119(AU)**
Inventor: **WONG, Michael, Su, Fah**
**5 Dnignan CLose**
**Epping, NSW 2121(AU)**
Inventor: **PAULL, David, Robert**
**11 Robina Crescent**
**Armidale, NSW 2350(AU)**

(74) Representative: **Daley, Michael John**
**F.J. CLEVELAND & COMPANY**
**40/43 Chancery Lane**
**London, WC2A 1JO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 316 320 B1

**Description**

This invention concerns methods and immunogenic mixtures for reducing ova and embryo losses in groups of domestic animals such as sheep, goats, pigs, cattle, deer, llamas and alpacas, which groups are hereinafter called "flocks".

Because of the considerable economic importance of domestic livestock, studies have long been directed toward processes by which the individual fecundity of farm animals might be manipulated. In particular, attention has been given to enhancing fecundity by increasing ovulation rates as this is a major step influencing reproductive performance.

However, ovulation rates do no necessarily correlate with fecundity. In the animal production industry it is well known that some of the ova produced in a single oestrus period, even when fertilized, do not survive to become live offspring. In the case of sheep, for example, about 10% of single ovulations, about 25% of twin ovulations and about 35% of triple ovulations that occur do not result in lambs being born. When treatments to increase ovulation rate such as immunization against steroids as described in our Australian patent No. 542638 are used, the losses are higher and can be as much as double those encountered under natural circumstances and this has been of particular concern in the Merino ewe.

While it has been apparent that control of ova and embryo losses offers scope for improving productivity, effective and economic procedures for reducing such losses have not hitherto been available, because the mechanisms involved are complex and many factors are likely to be implicated. Thus, inadequacies or imbalances in hormone levels may adversely affect the normal pattern of the reproductive cycle and thereby affect an animal's reproductive tract. Losses may be associated with ova not reaching the fallopian tube, with failure to fertilize ova, unsatisfactory transport of fertilized ova to the uterus, inadequate uterine cyclical change or maintenance, delayed implantation, and failure to sustain and nourish the implanted embryo.

Hormonal factors are known to be closely involved in all these stages, but the various hormone levels, and the patterns during the oestrus cycle, vary from species to species and as yet have only been delineated in part. Although some success in increasing the survival rate of embryos has been achieved through artificially altering hormone levels by administration of steroids, and indirectly by nutritional manipulation, such prior art procedures are cumbersome, expensive, inconsistent and not widely applicable.

The present invention offers a more practicable and generally useful approach to improving livestock productivity, and its essence lies in the discovery that by immunizing an animal against several steroids simultaneously, through the use of specially prepared immunogens administered under defined conditions, an increased proportion of ova shed at a particular oestrus survive as live offspring delivered per pregnancy.

British patent specification 2,115,281 discloses the passive immunization of female animals with an antiserum to an androgen steroid and an antiserum to an oestrogen steroid for the purpose of increasing fecundity in the animals. The present inventors have surprisingly found that with the more difficult active immunization strategy ova and/or embryo loss may be reduced, and therefore fecundity increased, in female animals in a flock by the use of certain immunogenic androgenic and oestrogenic conjugates administered substantially simultaneously prior to a mating period and so adjusting the active immunization regime that desired antibody titres are achieved during the mating period while ensuring that the titres decline at a preferred rate during that mating period. Such an approach is not suggested by the prior art which specifically teaches away from active immunization.

The present invention consists in a method for reducing ova and/or embryo losses and hence increasing the fecundity in a flock of domesticated animals comprising immunizing the female animals in the flock against a group of steroids, said group of steroids comprising one steroid from each of the classes of androgens and oestrogens, the immunization being prior to a mating period lasting at least twice the length of the average oestrus cycle for the species of animals concerned, whereby antibodies are present in the immunized female animals in the flock against each of the group of steroids simultaneously during the mating period at sufficient levels such that ova and/or embryo losses are reduced and fecundity is increased while being low enough not to reduce substantially the display of behavioral oestrus by the immunized female animals during the mating period and the immunization being such that the levels of the said antibodies decline during the mating period, preferably by from 50 to 80%.

More specifically, the method involves producing mean antisteroid antibody levels (titres) in the flock or herd against the various steroids within a specified range at the start of the mating period and maintaining the mean titres within the range over a mating period lasting at least twice the length of the average oestrus cycle relevant to the species concerned. This can most conveniently be done by using a specified system of immunogens, adjuvants and appropriate protocols which have been shown by pre-testing in a small

2

group of trial animals to produce the required antibody levels.

Also according to the present invention, we provide an immunogenic mixture for reducing ova and/or embryo loss and hence increasing fecundity when administered to females in a flock of domesticated animals, comprising immunogenic steroids protein conjugates capable of raising in the animals antibodies against a group of steroids, said group of steroids comprising one steroid from each of the classes of androgens and oestrogens, and an immunoadjuvant.

As used in this specification the following terms have the meanings set out hereunder:

Androgen

Any substance that stimulates the expression of secondary sex characteristics of the male. Androgenic activity can be assessed by measuring the regrowth of the involuted comb of a castrated cock following androgen administration or by measuring the growth response of the seminal vesicles in castrated male rate following androgen administration.

Anoestrus

The absence of oestrus or mating activity, whether or not ovulation occurs.

Antibody Titre

Defined here as the final dilution of the antiserum which binds 50% of the maximum amount of tritium labelled steroid bound by the antiserum during incubation with about 50 picograms of steroid in 0.4ml of phosphate buffer, 0.05M, pH 7.4 containing 0.1% porcine gelatin, 0.9% sodium chloride and 0.1% sodium amide for about 18 hours at 4°C followed by the use of either dextran-coated charcoal or polyethyleneglycol to separate free from antibody-bound steroid.

Thus, if 50% of the steroid is bound when the final serum dilution in buffer is 1 in 2000, this is the antibody titre. For comparisons of titres between test groups of at least six animals the mean titre of the group is expressed as the arithmetic mean.

Corticosteroids

Compounds secreted by the cortex of the adrenal gland which have widespread biological activity, including promotion of the synthesis of glycogen in the liver, and acceleration of catabolism of proteins. They also prevent inflammatory responses due to bacterial stimuli.

Drakeol® Oil Adjuvant

Such a preparation is typically 4 parts of purified paraffin oil containing about 11% Arlacel A surfactant to 1 part aqueous containing about 4.25% Tween® 80 surfactant. The immunogen and the oil-water mixture are ultrasonicated to form an emulsion.

Embryo

After fertilization the ovum initially divides to a 2-cell stage. In this specification embryo means the 2-cell and subsequent stages.

Fecundity

Is a measure of the number of lambs borne by a ewe and is defined as live lambs born per ewe.

Fertility and Fertility of a Flock

The ability of an animal to conceive and maintain a pregnancy. The fertility of a flock of sheep is assessed from the percentage of ewes which lamb following a 5-6 week period in which ewes and rams are permitted to breed. That percentage of ewes which lamb is the percentage of fertile ewes in the flock.

Flock Fecundity

Is used in the sense of the average number of live lambs born per ewe in the flock which are permitted to breed.

Hapten and Hapten Density

The hapten in this invention refers to the steroid substituent which is co-valently linked to the immunogenic protein molecule. Hapten density is a measure of the hapten incorporation into the protein carrier molecule following the chemical conjugation. The hapten density has been expressed as the average number of steroidal residues substituted per 100 amino acid residues in the protein structure in order to allow for variations in molecular weight of the proteins which can be used. Oestrogen

Any substance other than oestrodiol-17$\beta$ that stimulates the expression of secondary sex characteristics in the female. Oestrogenic activity can be assessed by measurement of uterine growth or cornification of the vaginal epithelium following oestrogen administration to spayed female rats or mice.

Ovulation Rate

The number of eggs released from the ovaries at ovulation. On a flock basis it is generally expressed as the number of eggs shed per ewe ovulating, and is therefore always equal to or greater than 1.

Primary and Booster Immunization

Primary immunization is the initial injection of immunogens given to stimulate antibody production to those immunogens. In this specification, a booster immunization is that given after the primary treatment. Thus the first booster is the treatment following the primary immunization and the second booster the next treatment again.

Progestrogen

Steroid compounds which complete the proliferation of the uterine endometrium after initiation by oestrogen, and which aid in the maintenance of pregnancy.

The process according to the present invention results, as would be expected from the prior art on the effects of immunization of sheep against single steroids, in a pattern of significantly increased ovulation rate that can be observed by laparoscopy and a gain in lambing rate. However, in the prior art a considerable proportion of ovulations do not yield lambs and this represents a loss of potential production.

The new process, in generating a particular balance of antibodies against several selected steroids simultaneously, surprisingly results in a considerable further gain in productivity due to higher proportion of ovulations producing offspring compared to that following immunization against individual component steroids. Moreover, it can be found that by using mixtures of androgen and oestrogen immunogens (preferably androstenedione, testosterone and oestrone) there is also a further gain in ovulation rate, and hence in lambing percentage above that from single immunogen treatment.

Further, the process according to the present invention results, as would be expected from the prior art, in antibody formation specific to a particular steroid (A) incorporated in a steroid-protein immunogen, but unexpectedly it is found that after the initial immunization with multiple steroids, antibody formation to steroid (A) can by some degree be elicited by immunization against another steroid (B) linked to an identical protein or even to a different protein. Moreover, by altering the steroids and the proteins in the immunogens used at any given treatment it has even been found possible to manipulate the antibody responses to selected steroids, so obtaining control over the relative concentrations of antibody formed specific to particular steroids. A further surprising discovery is that particular ratios of antibodies against various steroids are required to produce optimal biological responses, i.e. increased offspring with reduced ova and embryo loss.

The immunizations of the invention are performed by using conjugates of steroidal androgen, oestrogen, and optionally progestrogen or corticosteroid, with immunogenic proteins.

The average flock or herd titre for a particular steroid that is obtained and maintained (i.e. the time-response curve) is essentially controlled by the nature of the mixture of immunogens used in the primary and booster immunizations (which mixtures can be different), by the nature of the protein carriers used for each immunogen, by the nature of the immunoadjuvant used, by the time elapsing between the primary and first booster immunization and by the hapten density.

The mixture of immunogens is selected from the steroid classes of androgen and oestrogen, with progestrogen or corticosteroid as optional additions linked to proteins such as human or bovine serum albumin, ovalbumin lactalbumin, gelatin, casein, thyroglobulin, $\gamma$ -globulin etc. The invention places no limitation on the protein used to form the immunizing antigen of the invention, there being many examples in the prior art of suitable protein which when conjugated to the steroid will produce the required antibody responses.

The steroids may be linked to separate protein carriers, each steroid-protein conjugate being a separate immunogen, and subsequently injected into the animal as a physical mixture. However, for some combinations it may be satisfactory to have two or more steroids on the same molecule forming one immunogen capable of eliciting antibodies to the two or more distinct steroids.

According to this invention, the multiple antibody response to several steroids requires that immunogens effective for at least two steroids be used, one being an androgen and the other an oestrogen. The optimum mixture is dependent on the breed and species of animal to be treated, and the level of control which it is desired to elicit.

Thus, for example in Merino ewes improved ovulation rates and a lesser improvement in fecundity have been attained following immunization to a single steroid. However, optimum responses for lambs born/ova shed require antibodies to androstenedione, testosterone and oestrone to be formed in proportions specified subsequently. Some strains of Merino may respond with advantage to progesterone or corticosteroid antibodies in addition to androgen and oestrogen antibodies. In general for merino ewes, androstenedione, testosterone and oestrone chemically linked to protein carriers are required for the primary immunization. However, for the booster immunizations, surprisingly, the use of androstenedione-protein and/or testosterone-protein without an oestrone conjugate usually suffices. Such effects allow for the control of the oestrone titre to a lower value than that for the androgens which has been found to be desirable. To control the level of response to oestrone a different preferably less immunogenic carrier can also be used for this steroid in contrast to the carrier used for the androgens, resulting in a weaker antibody response to oestrone being elicited on further immunization.

Thus it has been found useful in Merino ewes to give a primary immunization of testosterone-3CMO-human serum albumin together with androstenedione-7$\alpha$ CETE-human serum albumin mixed with oestrone-3CM-bovine serum albumin, at a dosage of about 0.5 - 2.0mg of each immunogen per animal. The first booster immunization is then given only with either androstenedione:HSA or testosterone:HSA at a similar dosage. In the primary response similar levels of antibodies to testosterone, androstenedione and oestrone form, but after the secondary response (although all antibody levels increase) oestrone antibody concentrations rise less than those of the androgens.

By immunizing at the booster treatments either with a single antigen directed against one steroid, or against various combinations of antigens directed against two or more steroids, different proportions of steroid reactive antibodies are formed in the animal. We have found by experimentation a number of general features of these responses with steroid-protein immunogens in sheep. Immunization with a mixture of several steroid-protein immunogens in DEAE-dextran adjuvant may lead to lower antibody titres than any of the immunogens would produce separately. The most immunogenic compound in the mixture tends to produce the highest titre. Following primary treatment with a mixture of immunogens, further treatment with only a single immunogen results in the antibody titre for the steroid of that immunogen rising most strongly. If the protein carriers of the other steroid immunogens are the same as that in the immunogen used for boosting then the antibody levels for these steroids also rise well, whereas those having a different carrier are associated with lesser responses.

As an alternative approach to controlling the oestrone titre, it has been found that the relative proportions of androgen and oestrogen immunogens in the immunization mixture may be altered to produce the desired antibody response. Thus Merino ewes treated in the primary and subsequent immunizations with equal amounts of androstenedione-7$\alpha$ -CETE-human serum albumin and oestrone-3CM-human serum albumin show a higher antibody titre for oestrone than androstenedione. Reduction in the amount of oestrone immunogen relative to androgen immunogen in the mixture favours the production of higher levels of androgen antibody with a weaker oestrone antibody responses.

As a further approach, two or three of the steroids may be conjugated to one protein carrier in such proportions as to produce the antibody responses required in the immunization of sheep. Thus androsteredione and oestrone can be conjugated by simultaneous or sequential reaction to human serum albumin, with judicious selection of reagent concentrations, so as to produce the desired immunogen with the required ratio of the two haptens.

A high oestrogen to androgen antibody ratio has been found to be of disadvantage, resulting in embryo loss or fertilization failure, and even loss of oestrus behaviour.

In Merino ewes, it has been found an advantage to allow 14-42 days (optimally 21-28 days) interval between the primary and first booster and then to allow 3-7 weeks (optimally 4 to 5 weeks) to elapse after the booster immunization and before the rams are placed with the ewes for a normal mating period of 5-6 weeks. This interval of 3-7 weeks allows antibody titres to decline to the desired range and the endocrine system to stabilize. The range of desired antibody titres as an average value for a flock at the start of the 5-6 week mating period is shown in the following Table.

| Antibody Titres at the Start of Mating for Merino Ewes - To Increase Ovulation Rate and Maximise Lambing Percentages | |
| --- | --- |
| | Average Antibody Titre Range |
| Androstenedione ) | 1:100 - 1:3000 |
| Testosterone ) | 1:100 - 1:3000 |
| Oestrone ) | 1:100 - 1:0500 |
| Total androgen titre | 1:250 - 1:4000 |

Optimal Ratio of Oestrogen
titre to total androgen titre Below 3:4

| Optionally: | |
| --- | --- |
| Progesterone ) | 1:50 - 1:500 |
| Cortisol ) | 1:50 - 1:500 |

As these critical antibody titre ranges are exceeded, flock fecundity declines to normal and eventually to subnormal levels as a progressively greater proportion of animals fail to display oestrus in consequence of the immunization. With mean antibody titres which are significantly lower than these ranges, flock fecundity will approach that characteristic of unimmunized animals.

There is no need for antibody titres to be measured in each flock treated. It is sufficient if the system used, including immunogens, adjuvant, and the appropriate protocol is pre-tested in a small group of female animals of about 7-10 in number and is shown to produce the required titres. If necessary, further modifications of immunogens as indicated previously can be used in order to adjust responses.

Thus, by simple experimentation it is possible to derive suitable immunization systems, within the concepts of this invention, that can be defined as effective by the testing of the system in small group of female animals. These immunization systems including the pre-tested immunogen mixture can then be applied to large flocks of animals to produce the specified improved biological effects without further antibody titre measurements.

The invention recognizes that the critical range of antibody titres will vary with the hormone used in the immunization and with the breed and strain of sheep being immunized. The critical titre range given in the foregoing examples are cited purely by way of example and not for the purpose of setting precise limitations on the critical antibody titre range for all possible hormone immunizations. In general, the greater the oestrogenic immunizing oestrogen the lower will be the general level of the critical oestrogen antibody titre and the narrower will be its range. Similar variations in response according to breed may be expected with other species.

Variations in the range of antibody titres using mixtures of steroid immunogens can be used to produce three advantageous situations superior to that resulting from immunization against a single immunogen. At low antibody titres, with no increase in the natural ovulation rate, there can be a gain in the offspring born because of better ova and embryo survival. At higher antibody titres there is an increase in ovulation rate similar to that with immunization against a single steroid, but the new process results in a higher ratio of offspring born per ova shed. Further, under ideal conditions the ovulation rate is higher than that with single steroid immunization and hence an even greater number of offspring are born through both increased ovulation rate and reduction of ova and embryo loss.

The formed antibodies that are directed to each steroid (i.e. relate to each hapten) should be highly specific. Each steroidal hapten individually should produce specific antibodies in order to have a precise control over the biological effects, even though, according to this invention, 2 to 5 steroidal haptens may be mixed and used.

A special case is that in which a single hapten, by being linked to the protein through a particular substituent position can result in an immunogen which is effective in producing antibodies to 2 steroids. Such an example is testosterone -17- hemisuccinate which as a hapten results in antibodies of similar reactivity to both testosterone and androstenedione.

In preparation of the immunogens, substances already known in the art may be used. Thus preferred immunogens are prepared by the conjugation to protein of 4-androstene-3,17-dione derivatised with an acid group at positions 1, 3, 7, 6, 11 or 15, 17 of the steroid ring for example.

Preferred immunogens for testosterone are prepared by the conjugation to protein of testosterone derivatised with an acid group at 1, 3, 6, 7, 11, 15 or 17 of the steroid ring for example.

The preferred oestrone steroid derivatives are those which after conjugation with protein and administration to an animal, produce oestrone-specific antibody with little cross-reaction with oestradiol and are prepared by the conjugation to protein of oestrone with an acid group at 3, 6, 11, 15, 16, 17 of the steroid ring for example.

Non-limiting examples of such derivatives already known in the art include 4-androstene-3, 17-dione- $7\alpha$ -carboxyethyl thioether, 4-androstene-6$\beta$ -ol-3, 17-dione-hemisuccinate, 4-androstene-3, 17-dione-3 - carboxymethyloxime, testosterone-17-hemisuccinate, testosterone-3-carboxymethyloxime, oestrone-3-hemisuccinate, oestrone-3-carboxymethyl ether, oestrone-6-carboxymethyloxime and 15-carboxymethyloestrone.

For progesterone the non-limiting examples are derivatives of progesterone derivatised in the 11, 3, 6 or 7 positions of the steroid ring. Similar derivatisations of cortisol are at positions 3, 6, 7, 11, 21 of the steroid for example.

The invention places no limitation on the protein used to form the immunizing antigen of the invention.

For all the above immunogens, and using various methods known in the prior art for their synthesis, it is nevertheless required to prepare immunogens which can adequately stimulate antibody formation. This requires that the immunogens be synthesised with preferred hapten densities. The hapten density of immunogens, where each steroid is conjugated to separate protein molecules, is preferably from 3.1 to 5.2 for each of the androgens and from 1.7 to 3.8 for oestrone, provided the hapten density of the oestrone conjugate is always less than that of either androgen. When immunogens are synthetised with two or more steroids on the same protein molecule, the desired hapten density is, surprisingly, very low for the oestrogen. Thus for each androgen derivative the hapten density is preferably from 3.4 to 5.2, whereas that for oestrone is preferably from 0.17 to 1. However, slight variations in hapten density can be accommodated by varying the time period between primary and first booster immunizations, or by varying the adjuvant. Once batches of immunogen have been prepared they should be injected, together with a suitable adjuvant, preferably DEAE-dextran, into small number of test animals and the antibody titres of those test animals regularly observed over a period. This simple experimentation will reveal the potency of the immunogens and indicate their ability to produce antibody titres within the desired ranges. Further such small tests should be carried out with selected mixtures of immunogens to be used and if necessary, further modifications of immunogens as indicated previously can be made to modify responses before their application to large numbers of animals. The general magnitude of the mean antibody titres and their ranges are a function of the hormones used for immunization, and the antigenic competition of both steroid haptens and carrier proteins. When pretesting has indicated that the selected immunogens, adjuvant and protocol are suitable, flock treatment can proceed without the need for further antibody titre measurements.

As a matter of practical flock management it is not possible to ensure that a given sheep is mated and fertilized in any one oestrus cycle. It is therefore an essential feature of this invention that the immunization be carried out in such a way that the desired antibody titre in the flock is within effective levels for a mating period of at least five weeks (at least twice the length of the average oestrus cycle). Since, with the preferred procedures and adjuvants the antibody titres decline rapidly with time, at the end of mating they should be substantially (50-80%) of those at the start.

This invention is also of considerable importance to maiden ewes, as well as to mature ewes. For maiden ewes the use of immunization against several steroids as herein described minimises the loss of ova and embryos that often occurs during the first mating season.

The invention is not limited to effects on Merino ewes. With other breeds and cross-bred animals, whilst optimal antibody levels and ratios may vary somewhat, similar immunizations simultaneously against several steroids and proteins are also embraced by the concepts and processes of this invention. For other domestic species such as pigs, goats, cattle, deer and other exotic breeds like immunizations to achieve the objects of the invention are embraced by its concepts and processes. The invention is of particular importance to the pig industry, since the gilt and sow typically have an average ovulation rate of about 14, but will waste about half of these in embryo losses. Pigs have been found to respond similarly to sheep in their production of antibody titres following immunization to steroid/protein conjugates.

To develop an antibody response to the steroid haptens of steroid-protein conjugates it is necessary to perform immunization with such conjugates using the aid of an immunoadjuvant, of which there are many well known in the art but of which Freund's adjuvant is probably the best known. Freund's adjuvant can be used with the protein antigens embraced by the invention to produce the gains in productivity described, but in doing so it will bring about the formation of granulomatous lesions in the tissues of the immunized livestock and to avoid this feature of Freunds adjuvant, another mineral oil, such as Drakeol®, or an aqueous based polyionic adjuvant such as DEAE-dextran, and styrene maleicanhydride copolymers may be used to potentiate the humoral antibody responses of the invention. The preferred method uses DEAE dextran which does not produce the undesirably strong local tissue reactions that are frequently characteristic of Freund's adjuvant, and is more readily controlled to give desirable titre responses and suitable titre decline curves with time. Suitable antibody responses, increased ovulation rates and reduced ova and embryo losses may also be achieved with the aid of Drakeol® oil adjuvant. Moreover, a second immunization is not required as suitable antibody titres are attained by about 4 weeks when joining may commence.

The following examples are given to illustrate preferred methods within the broad scope of this invention.

Example 1 Preparation of Steroid-Protein Immunogens

(a) To 3.6g testosterone-3-carboxymethyloxime dissolved in 240ml dioxane was added, dropwise and with stirring over a period of 5 minutes, a freshly prepared solution of 1.36g 1-ethyl-3 (3-dimethylaminopropyl) carbodiimide hydrochloride (ECDI) dissolved in 84ml distilled water. The reaction was allowed to proceed for 30 minutes at 25°C, and then a solution of 3.6g bovine serum albumin (BSA) dissolved in 160ml phosphate buffer ph 7.80, 0.1M was added dropwise with stirring. The reaction was allowed to continue for 24 hours, and then a further 50ml phosphate buffer and 1.1g ECDI in 20ml water was added with stirring.

After a further 4 hr at 25°C the reaction mixture was transferred to dialysis tubing and dialysed against distilled water. The water was changed after 2, 4, 22, 72 and 96 hr. The product (testosterone - 3 CMO : BSA) retained in the dialysis sack was then lyophilised and weighed. Yield 4.15g.

The steroid content of the steroid-protein immunogen was calculated by incorporating a trace amount of $^3$H-labellled testosterone-3-carboxymethyloxime in the reaction. Thus from the specific activity of this steroid derivative and of the steroid-protein immunogen, both determined by liquid scintillation counting of weighed amounts of the compounds, the number of moles steroid/mole BSA was obtained and this was then expressed as hapten density.

In the present preparation a hapten density of 3.8 (22 moles steroid/mole BSA) was obtained.

In similar preparations, 3-carboxymethyl-oestrone was conjugated to human serum albumin (HSA) and yielded oestrone-3CM:HSA with a hapten density of 3.4 (21 moles steroid/mole HSA), and androstenedione - 7α -carboxyethylthioether (androstenedione - 7α CETE) was conjugated to human serum albumin with a hapten density of 3.7.

The hapten density can be varied readily by using a different proportion of steroid derivative (together with the corresponding amount of ECD1) to the protein in the reaction.

Careful control of hapten densities and a high efficiency of steroid reacted can also be achieved conveniently by the use of active ester derivatives such as the N-hydroxy succinimide esters of the steroid acids as has been described in prior art.

(b) Preparation of an Immunogen Containing both Androgenic and Oestrogenic Haptens

To 20 mg. 3-carboxymethyl-oestrone dissolved in 16ml dioxane was added, dropwise and with stirring over a period of 5 minutes, a freshly prepared solution of 12mg. ECDI dissolved in 4.8 ml distilled water. The reaction was allowed to proceed for 30 minutes at 25°C, and then a solution of 60mg androstene-dione-7α CETE:HSA (prepared as in (a)) dissolved in 17 ml. phosphate buffer pH 7.80, 0.1M was added dropwise with stirring. The reaction was allowed to continue for 24 hours. The reaction mixture was then transferred to dialysis tubing and dialysed against distilled water. The water was changed after 2, 4, 22, 72 and 96 hr. The product retained in the dialysis sack was then lyophilised and weighed. Yield 65 mg. of androstenedione-7α CETE/oestrone-3CM:HSA with a hapten density of 4.5 androstenedione residues and 0.5 oestrone residues/100 amino acid residues.

Example 2 Immunization of Sheep to Test the Response to Several Steroid Immunogens

(a) For each immunogen (androstenedione-7α CETE:HSA, testosterone-3CMO:BSA and oestrone-3CM:HSA) 84 mg was pasted in 0.2ml 0.9% sterile saline and made up to a volume of 70ml 0.9% sterile saline. Then 70ml DEAE-dextran solution was added; this solution was prepared by dissolving 15g DEAE-dextran in 100ml water, bringing the pH to 7.5-7.7 using saturated tri-(hydroxymethyl)-methylamine buffer (500g/litre water) , and adjusting the final volume to 150ml.

Fifty sheep were each injected with 2ml of each immunogen solution; each sheep received 1ml of androstenedione-7α CETE:HSA subcutaneously into two sites on the right side of the neck, 1ml of testosterone-3CMO:BSA subcutaneously into two sites on the left side of the neck and 2ml oestrone-3CM:HSA subcutaneously into the rump.

A second group of fifty sheep were each injected with 2ml androstenedione-7α CETE:HSA only, each sheep being given 1ml subcutaneously into two sites on the right side of the neck.

Twenty one days later, the same injection procedures were repeated. However, the first group of sheep were injected with only two of the three immunogens, namely androstenedione-7α CETE:HSA and testosterone-3CMO:BSA in saline-DEAE dextran.

Blood samples were taken by venepuncture two weeks after the first injection and 1, 5 and 9 weeks following the second series of injections. Blood was collected into heparinized tubes, stored on ice and centrifuged at 4°C. The plasma so obtained was stored at -10°C before steroid-antibody titre determination.

The antibody titres obtained following injection with multiple immunogens and with androstenedione-7α CETE:HSA alone are shown in Table 1.

The multiple immunogens do not have to be injected separately. Equally effective immunizations can be achieved, for example, by subcutaneous injection of 2ml of a vaccine prepared by combining and pasting together 84mg of each immunogen, and making up to a final volume of 70ml 0.9% sterile saline solution prior to adding 70ml DEAE-dextran solution.

(b) A mixture of 18mg each of androstenedione-7α CETE:HSA, testosterone-3CMO:HSA and oestrone-3CM:BSA was pasted in 0.2ml 0.9% sterile saline, and made up to a volume of 15ml sterile saline; to this was then added 15ml DEAE-dextran solution. Ten sheep were each injected with 2ml of the mixture subcutaneously in the neck. Three weeks later, the sheep were each given a second immunization of 2ml androstenedione-7α CETE:HSA only in DEAE-dextran (18mg in 15ml sterile saline and 15ml DEAE-dextran) administered in like fashion. In blood samples taken one, four and nine weeks after the second treatment, desirable levels of antibodies to all three steroids had been achieved. The results are shown in Table II.

Example 3

Immunization of merino ewes to increase ovulation rate and lambing percentage

(a) Fine wool merino ewes (150) aged 3 years were randomly allocated to (i) a control group, (ii) an androstenedione-7α CETE:HSA immunized group, and (iii) a group immunized against several steroid immunogens, namely androstenedione-7α CETE:HSA, testosterone-3CMO:BSA, and oestrone-CM:HSA.

The immunogens and the treatment were as described in Example 1a and 2a. The control group received no treatment. The ewes were mated with rams, the three groups being kept together as one flock and mating was continued during the period 4-10 weeks after completion of the immunization.

The occurrence of oestrus and mating was monitored, and the ovulation rate was observed by laparoscopy on the sheep within a week of oestrus being recorded. Lambs were recorded and identified with their mothers on the day of birth.

The following year, both immunized groups were retreated with 2ml androstenedione-7α CETE:HSA only in DEAE-dextran. In group (III) desirable levels of antibodies to all three steroids were achieved as shown by blood samples taken 1, 4 weeks after the booster treatment (Table III).

The results are shown in Table IV, and indicate that in the first year both treatments increased ovulation rate significantly without loss of oestrus. However, not all ovulations resulted in lambs, and only the group treated with the three steroid immunogens showed a significantly increased lambing percentage compared to the control animals.

Both immunization treatments resulted in only a slight increase in the number of dry ewes (Table V). Of the ewes lambing, 15% of the ovulations failed to produce offspring in the androstenedione-immune group, while only 6.4% were lost in the multiple-steroid immune group.

9

The increase in the total number of lambs born was considerably less in the androstenedione immune group, due largely to embryo losses (Table V). On the other hand the multiple-steroid immune group showed a good increase with much fewer embryo losses. The increase in lambs born was significantly greater in the multiple-steroid immune group than in either the control or the androstenedione immune group.

In the second year, both immunized groups showed an increase in ovulation rate and an increased percentage of foetuses compared to the control group. Again, the multiple-steroid immunized group had a greater percentage of ova surviving compared to the single steroid immunized group.

The poor lambing response in the androstenedione-immune ewes in the first year reflects that sometimes seen under field conditions with Merino ewes, and was clearly overcome by multiple-steroid immunity. The improved performance of ewes with multiple-steroid immunity was also maintained in a second year of treatment.

(b) A flock of 634 merino ewes, comprising 189 ewes in their first reproductive season and the balance being $2^1/2$ to 7 years old, were allocated to (i) a control group, (ii) an androstenedione-7α CETE:HSA immunized group and (iii) a group immunized against androstenedione-7α CETE:HSA, testosterone-3CMO:BSA, and oestrone-3CM:HSA.

The second immunization was given 22 days after the primary, group (iii) being treated with only two of the three initial immunogens, namely androstenedione-7α CETE:HSA and testosterone-3CM:BSA. The ewes were mated and lambed as described in Example 3a, but laparoscopy was restricted to a small number of ewes from each group.

The reproductive performance of the ewes (Table VI) was similar to that in Example 3a. Oestrous behaviour was not affected and both treatments increased the ovulation rates and lambing percentages compared to the control ewes. Again in these Merino ewes, there were fewer ova and embryo losses in the multiple-immune ewes than in the androsteredione immune ewes, and hence a significantly better lambing response following multiple-steroid immunization.

The improved fecundity of sheep following steroid immunization is further enhanced by the use of multiple rather than single steroid immunogens due to fewer ova and embryo losses.

TABLE I

<u>Antibody titres to steroids</u> (mean values for groups of 10 sheep)

| IMMUNOGENS USED | | | TIME OF BLOOD SAMPLE | | | |
|---|---|---|---|---|---|---|
| 1ST TREATMENT | 2ND TREATMENT | ANTIBODY TITRE TO | AFTER 1ST TREATMENT 2WK | AFTER 2ND TREATMENT | | |
| | | | | 1WK | 5WK | 9WK |
| **Group 1:** | | | | | | |
| Androstenedione-<br>7 $\alpha$ CETE:HSA | Androstenedione-<br>7 $\alpha$ CETE:HSA | Androstenedione | 1:35 | 1:820 | 1:240 | 1:80 |
| Testosterone-<br>3CMO:BSA | Testosterone-<br>3CMO:BSA | Testosterone | | 1:500 | 1:150 | 1:110 |
| Oestrone-3CM:HSA | − | Oestrone | 1:230 | 1:1100 | 1:280 | 1:110 |
| **Group 2:** | | | | | | |
| Androstenedione-<br>7 $\alpha$ CETE:HSA | Androstenedione-<br>7 $\alpha$ CETE:HSA | Androstenedione | nil | 1:2000 | 1:440 | 1:130 |

EP 0 316 320 B1

TABLE II

Antibody titres to steroids (mean values for a group of 10 sheep )

IMMUNOGENS USED IN

| 1ST TREATMENT | 2ND TREATMENT | TO | ANTIBODY TITRE AFTER 2ND TREATMENT | | |
|---|---|---|---|---|---|
| | | | 1WK | 4WK | 9WK |
| Androstenedione- 7$\alpha$CETE:HSA | Androstenedione- 7$\alpha$CETE:HSA | Androstenedione | 1800 | 630 | 150 |
| Testosterone- 3CMO:BSA | − | Testosterone | 3000 | 920 | 260 |
| Oestrone-3CM:HSA | − | Oestrone | 1400 | 430 | 110 |

## TABLE III

Antibody titres to steroids - 2nd Year Response.

(mean values for groups of 10 sheep)

| Initial Treatment | 2nd Year Treatment | Antibody Titre | |
|---|---|---|---|
| | | 1WK | 4WK |
| **Group ii** | | | |
| Androstenedione -7α CETE:HSA | Androstenedione -7 α CETE:HSA | 1:5000 | 1:2200 |
| **Group iii** | | | |
| Androstenedione -7 α CETE:HSA | Androstenedione -7 α CETE:HSA | 1:1800 | 1:610 |
| Testosterone - 3CMO:BSA | | 1:1400 | 1:600 |
| Oestrone - 3CM:HSA | | 1:690 | 1:230 |

EP 0 316 320 B1

TABLE IV

Responses in merino ewes to immunization to a single or several steroids

| GROUP | NO OF EWES JOINED | OVULATION RATE | % OESTRUS | 0 | 1 | 2 | 3 | 0 | 1 | 2 | TOTAL OVULATIONS | LAMBS | % LAMBS BORN EWES JOINED |
|---|---|---|---|---|---|---|---|---|---|---|---|---|---|
| | | | | | OVULATIONS | | | | LAMBS | | | | |
| **1st Year** | | | | | | | | | | | | | |
| Control | 50 | 1.04 | 100 | – | 48 | 2 | – | 4 | 43 | 3 | 52 | 49 | 98 |
| Androstenedione immunized | 50 | 1.32 | 100 | – | 35 | 14 | 1* | 5 | 39 | 6 | 66 | 51 | 102 |
| Multiple-steroid immunized | 50 | 1.38 | 100 | – | 31 | 19 | –* | 6 | 29 | 15* | 69 | 59 | 118 |
| **2nd Year** | | | | | | | | | | | | | |
| Control | 41 | 1.12 | 95 | 0 | 36 | 5 | – | 5 | 31 | 5+ | 46 | 41 | 100 |
| Androstenedione immunized | 43 | 1.37 | 98 | 1 | 27 | 13 | 2 | 7 | 22 | 14+ | 59 | 50 | 116 |
| Multiple-steroid immunized | 46 | 1.26 | 96 | 2 | 30 | 14 | – | 5 | 27 | 14+ | 58 | 55 | 120 |

\* Fischers exact test compared with controls P<0.01.

+ Foetuses present at 70-90 days gestation.

TABLE V

<u>Embryo loss in Merino ewes after immunization with a single or multiple steroid immunogen</u>

| | | | STEROID IMMUNIZATION TREATMENT |
| | Control | Androstenedione | Androstenedione Testosterone Oestrone |
| --- | --- | --- | --- |
| Ewes treated | 50 | 50 | 50 |
| % Showing oestrus | 100 | 100 | 100 |
| Dry ewes | 4 | 5 | 6 |
| Ewes lambing | 46 | 45 | 44 |
| Ovulations of all ewes at conception or final cycle | 52 | 66 | 69 |
| Ovulations of all ewes lambing | 48 | 60 | 63 |
| Lambs born | 49 | 51 | 59 |
| Lambs born/ova shed in all ewes in flock | 0.94 | 0.77 | 0.86 |
| Embryos lost | 0 | 9 | 4 |
| % Wastage rate | 0 | 15.0 | 6.4 |

EP 0 316 320 B1

TABLE VI

Responses in Merino ewes (1 1/2 - 7 yrs old) to immunization to a single or several steroids

| GROUP | No. of ewes joined | Ovulation Rate (No. ewes examined) | % Oestrus | No. of ewes with lambs | | | | Total lambs | % lambs born per ewe joined |
|---|---|---|---|---|---|---|---|---|---|
| | | | | 0 | 1 | 2 | 3 | | |
| Control | 124 | 1.10 | 94 | 28 | 90 | 6 | - | 102 | 82 |
| Androstenedione immunized | 254 | 1.42 | 93 | 81 | 125 | 48 | - | 221 | 87 |
| Multiple-steroid immunized | 256 | 1.45 | 95 | 56 | 148 | 51 | 1 | 253 | 99* |

* Significantly different to androstenedione-immunized ewes, $p < 0.05$.

## Claims

1. A method of reducing ova and/or embryo losses and thus increasing the fecundity in a flock of domesticated animals, characterised by:
   actively immunizing the female animals in the flock against a group of steroids including at least

16

EP 0 316 320 B1

one androgen and at least one oestrogen by treating said female animals with one or are conjugates of said steroids with one or are immunogenic proteins, there to procure an antibody response by each of said female animals simultaneously against each of the steroids prior to a mating period at least twice as long as the oestrus cycle of the animals concerned;

and thereafter sting the animals during said mating period;

the method being such that the levels of antibodies in the immunized female animals in the flock during the mating period are such that ova and/or embryo losses are reduced and fecundity is increased and are low enough not to reduce substantially the display of behavioural oestrus by said female animals, and the levels of said antibodies decline during the mating period.

2. A method as claimed in claim 1 in which the group of steroids includes one or more progestogens and/or one or more corticosteroids.

3. A method as claimed in claim 1 or 2 wherein the levels of the antibodies in said animal decline by 30 to 90% during the mating period.

4. A method as claimed in claim 1, claim 2 or claim 3, wherein the levels of the antibodies in said immunized female animals decline by 50% to 80% during the mating period.

5. A method as claimed in any of claims 1 to 4 in which the group of steroids comprises 4-androstene-3,17-dione, testosterone and oestrone.

6. A method as claimed in any of claims 1 to 5 wherein said immunogenic proteins are selected from human serum albumin, bovine serum albumin, ovalbumin, gelatin, casein, lactalbumin, thyroglobulin and $\alpha$-globulin.

7. A method as claimed in any preceding claim wherein the steroid-protein conjugates are administered together with an immunoadjuvant selected from diethylaminoethyl-dextran and Drakeol emulsion.

8. A method as claimed in any preceding claim wherein the immunization is performed using one or more immunogenic proteins each of which is derivatised with two or more different steroids as haptenic groups.

9. A method as claimed in any preceding claim wherein each steroid protein conjugate has a hapten density of from 3.1 to 5.2 where the steroid is an androgen and from 1.7 to 3.8 where the steroid is an oestrogen; provided the hapten density of the oestrogen conjugate is always less than that of the androgen conjugate.

10. A method as claimed in any preceding claim wherein the immunization is effected using a single steroid protein conjugate having a hapten density of from 3.4 to 5.2 in respect of steroidal androgen and from 0.17 to 1 in respect of steroidal oestrogens.

11. A method as claimed in any preceding claim wherein the said female animals are given a first active immunization against each of the steroids, and are subsequently given a further active immunization against the steroids prior to the start of the mating period.

12. A method as claimed in any preceding claim wherein the animals are sheep and the group of steroids includes one or two androgens and one oestrogen; the immunizations are carried out such that the mean steroid-binding antibody titres in the flock for each androgen is from 1 in 100 to 1 in 3000, and for oestrogen is from 1 in 100 to 1 in 1500 and is less than 75% of the total androgen titre at the start of the mating period; and the overall decline in mean steroid-binding antibodies in the female sheep does not exceed 80% during a mating period lasting 5 weeks.

13. A method as claimed in any preceding claim wherein the mean steroid-binding antibody titres in the flock for each androgen is from 1 in 200 to 1 in 2000, and for the oestrogen is from 1 in 200 to 1 in 1000 and is less than 66.6% of the total androgen titre.

17

**14.** An immunogenic mixture for reducing ova and/or embryo loss and hence increasing fecundity when administered to a female domesticated animal which mixture comprises an immunoadjuvant and one or more immunogenic steroid-protein conjugates capable of provoking an antibody response in the animal against a group of steroids which includes at least one androgen and at least one oestrogen.

**Patentansprüche**

**1.** Verfahren zur Verminderung von Eizellen- und/oder Embryoausfällen und somit zur Erhöhung der Fruchtbarkeit in einer Haustierherde, gekennzeichnet durch aktive Immunisierung der weiblichen Tiere in der Herde gegen eine Gruppe von Steroiden, die mindestens ein Androgen und mindestens ein Östrogen einschließen, durch Behandlung jener weiblichen Tiere mit einem oder mehreren Konjugaten jener Steroide mit einem oder mehreren immunisierenden Proteinen, um dadurch gleichzeitig gegen jedes jener Steroide eine Antikörper-Reaktion in jedem besagtem weiblichen Tier vor einer Paarungszeit hervorzurufen, die mindestens doppelt so lang wie der Brunstzyklus der betroffenen Tiere ist, und anschließende Paarung der Tiere während jener Paarungszeit, wobei in dem Verfahren die Antikörper-Konzentrationen bei den immunisierten weiblichen Tieren der Herde während der Paarungszeit so hoch sind, daß Eizellen- und/oder Embryoausfälle vermindert und die Fruchtbarkeit erhöht werden, aber niedrig genug sind, um eine wesentliche Abschwächung des Brunstverhaltens jener weiblichen Tiere zu vermeiden, und wobei die Konzentrationen jener Antikörper sich während der Paarungszeit verringern.

**2.** Verfahren nach Anspruch 1, in dem die Steroidgruppe ein oder mehrere Progestogene und/oder ein oder mehrere Kortikosteroide einschließt.

**3.** Verfahren nach Anspruch 1 oder 2, worin die Antikörper-Konzentrationen jener Tiere während der Paarungszeit um 30 bis 90% abnehmen.

**4.** Verfahren nach Anspruch 1, Anspruch 2 oder Anspruch 3, worin die Antikörper-Konzentrationen jener immunisierten weiblichen Tiere während der Paarungszeit um 50% bis 80% abnehmen.

**5.** Verfahren nach einem der Ansprüche 1 bis 4, in welchem die Steroidgruppe 4-Androsten-3,17-dion, Testosteron und Östron einschließt.

**6.** Verfahren nach einem der Ansprüche 1 bis 5, worin jene immunisierenden Proteine unter Humanserumalbumin, Rinderserumalbumin, Ovalbumin, Gelatine, Casein, Lactalbumin, Thyroglobulin und $\alpha$-Globulin ausgewählt sind.

**7.** Verfahren nach einem der vorhergehenden Ansprüche, worin die Steroid-Proteinkonjugate zusammen mit einem unter Diethylaminoethyl-Dextran und Drakeol-Emulsion ausgewählten Immunoadjuvans verabreicht werden.

**8.** Verfahren nach einem der vorhergehenden Ansprüche, worin die Immunisierung mit einem oder mehreren immunisierenden Proteinen durchgeführt wird, von denen jedes mit zwei oder mehreren verschiedenen Steroiden as Haptengruppe derivatisiert ist.

**9.** Verfahren nach einem der vorhergehenden Ansprüche, worin jedes Steroid-Proteinkonjugat eine Haptendichte von 3,1 bis 5,2 aufweist, wenn das Steroid ein Androgen ist, und von 1,7 bis 3,8, wenn das Steroid ein Östrogen ist, vorausgesetzt, daß die Haptendichte des Östrogenkonjugats stets geringer als die des Androgenkonjugats ist.

**10.** Verfahren nach einem der vorhergehenden Ansprüche, worin die Immunisierung mit einem einzigen Steroid-Proteinkonjugat mit einer Haptendichte von 3,4 bis 5,2 bezüglich des steroidalen Androgens und von 0,17 bis 1 bezüglich des steroidalen Östrogens durchgeführt wird.

**11.** Verfahren nach einem der vorhergehenden Ansprüche, worin jene weiblichen Tiere eine erste aktive Immunisierung gegen jedes dieser Steroide und danach eine weitere aktive Immunisierung gegen die Steroide vor Beginn der Paarungszeit erhalten.

**12.** Verfahren nach einem der vorhergehenden Ansprüche, worin die Tiere Schafe sind und die Steroidgruppe ein oder zwei Androgene und ein Östrogen umfaßt, wobei die Immunisierungen so durchgeführt werden, daß die mittleren Steroid bindenden Antikörpertiter in der Herde 1 in 100 bis 1 in 3000 für jedes Androgen und 1 in 100 bis 1 in 1500 für Östrogen betragen und zu Beginn der Paarungszeit weniger als 75% des Gesamtandrogentiters ausmachen, und wobei die Gesamtabnahme der mittleren Steroid bindenden Antikörper in den weiblichen Schafen während einer 5-wöchigen Paarungszeit 80% nicht überschreitet.

**13.** Verfahren nach einem der vorhergehenden Ansprüche, worin die mittleren Steroid bindenden Antikörpertiter in der Herde von 1 in 200 bis 1 in 2000 für Androgen und von 1 in 200 bis 1 in 1000 für Östrogen betragen und weniger als 66,6% des Gesamtandrogentiters ausmachen.

**14.** Immunisierendes Gemisch zur Verminderung der Eizellen- und/oder Embryoausfälle und somit zur Erhöhung der Fruchtbarkeit bei Verabreichung an ein weibliches Haustier, wobei das Gemisch ein Immunoadjuvans und ein oder mehrere immunisierende, zur Hervorrufung einer Antikörperreaktion im Tier gegen eine Steroidgruppe, die mindestens ein Androgen und mindestens ein Östrogen einschließt, fähige Steroid-Proteinkonjugate umfaßt.

**Revendications**

**1.** Méthode de réduction des pertes d'oeufs et/ou d'embryons et par conséquent d'accroissement de la fécondité chez un troupeau d'animaux domestiques, caractérisée par :
l'immunisation active des femelles du troupeau contre un groupe de stéroïdes comprenant au moins un androgène et au moins un oestrogène, en traitant lesdites femelles avec un ou plusieurs conjugué(s) desdits stéroïdes et d'une ou plusieurs protéine(s) immunogène(s), afin de provoquer une production d'anticorps chez chacune desdites femelles simultanément contre chacun des stéroïdes avant un rut dont la durée est au moins deux fois la durée du cycle d'oestrus des animaux concernés ;
puis l'accouplement des animaux au cours dudit rut ;
la méthode faisant en sorte que les taux d'anticorps, chez les femelles du troupeau immunisées, au cours du rut permettent de réduire les pertes d'oeufs et/ou d'embryons et d'augmenter la fécondité, tout en étant suffisamment bas pour ne pas réduire nettement l'apparition d'oestrus comportemental chez lesdites femelles, et les taux desdits anticorps diminuent au cours du rut.

**2.** Méthode selon la revendication 1 dans laquelle le groupe de stéroïdes comprend un ou plusieurs progestogène(s) et/ou un ou plusieurs corticostéroïde(s).

**3.** Méthode selon la revendication 1 ou 2 dans laquelle les taux des anticorps chez ledit animal diminuent de 30 à 90 % au cours du rut.

**4.** Méthode selon la revendication 1, la revendication 2 ou la revendication 3, dans laquelle les taux des anticorps chez lesdites femelles immunisées diminuent de 50 % à 80 % au cours du rut.

**5.** Méthode selon l'une quelconque des revendications 1 à 4 dans laquelle le groupe de stéroïdes comprend la 4-androstène-3,17-dione, la testostérone et l'oestrone.

**6.** Méthode selon l'une quelconque des revendications 1 à 5 dans laquelle lesdites protéines immunogènes sont choisies parmi l'albumine sérique humaine, l'albumine sérique bovine, l'ovalbumine, la gélatine, la caséine, la lactalbumine, la thyroglobuline et l'$\alpha$-globuline.

**7.** Méthode selon l'une quelconque des revendications précédentes dans laquelle les conjugués stéroïdes-protéines sont administrés avec un immunoadjuvant choisi parmi le diéthylaminoéthyldextrane et l'émulsion Drakeol.

**8.** Méthode selon l'une quelconque des revendications précédentes dans laquelle l'immunisation est réalisée à l'aide d'une ou plusieurs protéine(s) immunogène(s) dont chacune est dérivée avec deux stéroïdes différents ou plus comme groupes hapténiques.

19

**9.** Méthode selon l'une quelconque des revendications précédentes dans laquelle chaque conjugué stéroïde-protéine a une densité d 'hapténes de 3,1 à 5,2 lorsque le stéroïde est un androgène et de 1,7 à 3,8 lorsque le stéroïde est un oestrogène ; à condition que la densité d ' haptènes du conjugué d'oestrogène soit toujours inférieure à celle du conjugué d'androgène.

**10.** Méthode selon l'une quelconque des revendications précédentes dans laquelle l'immunisation est réalisée à l'aide d'un seul conjugué stéroïde protéine dont la densité d 'haptènes est de 3,4 à 5,2 pour ce qui est de l'androgène stéroïdien et de 0,17 à 1 pour ce qui est des oestrogènes stéroïdiens.

**11.** Méthode selon l'une quelconque des revendications précédentes dans laquelle on administre auxdites femelles une première Immunisation active contre chacun des stéroïdes, puis une autre immunisation active contre les stéroïdes avant le début du rut.

**12.** Méthode selon l'une quelconque des revendications précédentes dans laquelle les animaux sont des moutons et le groupe de stéroïdes comprend un ou deux androgènes et un oestrogène ; les immunisations sont réalisées de façon que les titres moyens d'anticorps fixant le(s) stéroïde(s) chez le troupeau pour chaque androgène soient de 1 pour 100 à 1 pour 3000, et pour l'oestrogène de 1 pour 100 à 1 pour 1500 et soient inférieurs à 75 % du titre total d'androgéne au début du rut ; et la diminution globale des anticorps moyens fixant le(s) stéroïde(s) chez la brebis ne dépasse pas 80 % au cours d'un rut durant 5 semaines.

**13.** Méthode selon l'une quelconque des revendications précédentes dans laquelle les titres moyens d'anticorps fixant le(s) stéroïde(s) chez le troupeau pour chaque androgéne sent de 1 pour 200 à 1 pour 2000, et pour l'oestrogène de 1 pour 200 à 1 pour 1000 et sont inférieurs à 66,6 % du titre total d'androgène.

**14.** Mélange immunogène permettant de réduire la perte d'oeufs et/ou d'embryons et par conséquent d'accroître la fécondité lorsqu'on l'administre chez un animal domestique femelle, lequel mélange comprend un immunoadjuvant et un ou plusieurs conjugué(s) immunogène(s) stéroïde(s)-protéine(s) capable(s) de provoquer une production d'anticorps chez l'animal contre un groupe de stéroïdes qui comprend au moins un androgéne et au moins un oestrogène.